(19) Europäisches Patentamt
European Patent Office
Office européen des brevets

(11) Veröffentlichungsnummer: **0 372 665 A1**

# (12) EUROPÄISCHE PATENTANMELDUNG

(21) Anmeldenummer: **89250082.8**

(51) Int. Cl.[5]: **C07J 53/00, A61K 31/56**

(22) Anmeldetag: **10.11.89**

(30) Priorität: **11.11.88 DE 3838779**

(43) Veröffentlichungstag der Anmeldung:
**13.06.90 Patentblatt 90/24**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Kirsch, Gerald, Dr.**
**Luciusstrasse 6b**
**D-1000 Berlin 33(DE)**
Erfinder: **Laurent, Henry, Dr.**
**Glambeker Weg 21**
**D-1000 Berlin 28(DE)**
Erfinder: **Wiechert, Rudolf, Prof. Dr.**
**Petzower Strasse 8a**
**D-1000 Berlin 39(DE)**
Erfinder: **Beier, Sybille, Dr.**
**Uhlandstrasse 121**
**D-1000 Berlin 15(DE)**
Erfinder: **Elger, Walter, Dr.**
**Schorlemer Allee 12b**
**D-1000 Berlin 33(DE)**
Erfinder: **Bull, James R., Dr.**
**Waterkloof Ridge 2**
**342 Ridgeview Road Pretoria(ZA)**
Erfinder: **Neef, Günter Dr.,**
**Markgraf-Albrecht-Strasse 4,**
**D-1000 Berlin 15(DE)**

(54) **14alpha, 17alpha-Ethano-estratriene.**

(57) Es werden neue 14α,17α-Ethano-estratriene der allgemeinen Formel I

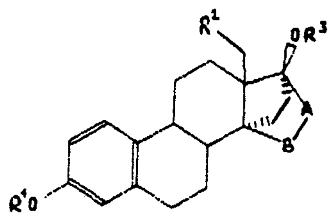

(I),

worin
$R^1$ ein Wasserstoffatom, eine Alkyl- oder Acylgruppe mit 1 bis 12 Kohlenstoffatomen,
$R^2$ ein Wasserstoffatom oder eine Methylgruppe,
$R^3$ ein Wasserstoffatom oder eine Acylgruppe mit 1 bis 12 Kohlenstoffatomen und

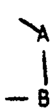 entweder 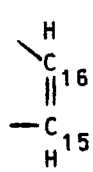 oder 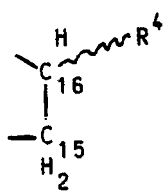

EP 0 372 665 A1

wobei $R^4$ ein $\alpha$- oder ß-ständiger Alkylrest mit 1 bis 8 Kohlenstoffatomen oder ein gegebenenfalls mehrere Doppelbindungen aufweisender $\alpha$,ß-Alkenyl- oder ein $\alpha$,ß-Alkinylrest, beide mit 2 bis 8 Kohlenstoffatomen, ist,
bedeuten, beschrieben sowie ein Verfahren zu deren Herstellung. Die neuen Verbindungen besitzen starke östrogene Wirksamkeit.

# 14α,17α-Ethano-estratriene

Die vorliegende Erfindung betrifft 14α,17α-Ethano-estratriene der allgemeinen Formel I

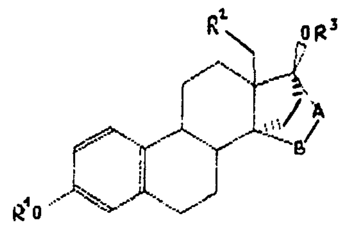

(I),

worin
$R^1$ ein Wasserstoffatom, eine Alkyl- oder Acylgruppe mit 1 bis 12 Kohlenstoffatomen,
$R^2$ ein Wasserstoffatom oder eine Methylgruppe,
$R^3$ ein Wasserstoffatom oder eine Acylgruppe mit 1 bis 12 Kohlenstoffatomen und

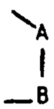 entweder 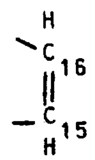 oder 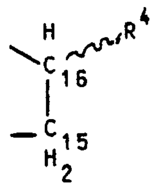

wobei $R^4$ ein α- oder ß-ständiger Alkylrest mit 1 bis 8 Kohlenstoffatomen oder ein gegebenenfalls mehrere Doppelbindungen aufweisender α,ß-Alkenyl- oder ein α,ß-Alkinylrest, beide mit 2 bis 8 Kohlenstoffatomen, ist,
bedeuten.

Die Erfindung betrifft außerdem ein Verfahren zur Herstellung der Verbindungen der allgemeinen Formel I, pharmazeutische Präparate, die Verbindungen der allgemeinen Formel I enthalten sowie die Verwendung der erfindungsgemäßen Verbindungen zur Fertilitätskontrolle der Frau sowie zur Behandlung von Estrogen-Mangelerscheinungen der Frau.

Als Acylgruppen $R^1$ und $R^3$ kommen Reste von organischen Carbonsäuren mit 1 bis 12 Kohlenstoffatomen infrage. Sie leiten sich ab von aliphatischen, cycloaliphatischen, aliphatisch-cycloaliphatischen, cycloaliphatisch-aliphatischen und aromatischen und aromatisch-aliphatischen Monocarbonsäuren. Die Anzahl der Kohlenstoffatome im Cycloaliphaten variiert von 3 bis 7, als Aromaten sind der Phenyl- sowie Naphtylrest zu nennen.

Als Reste $R^1$ und $R^3$ für die Acylgruppen werden der Essig-, Propion-, Butter-, Isobutter-, Pivalin-, Capron-, Acryl-, Croton-, Heptyl-, Capryl-, Pelargon-, Decan-, Undecan-, Dodecan-, 3-Cyclopentylpropion- und Benzoesäurerest bevorzugt.

Steht $R^1$ für eine Alkylgruppe, so kommen hier Alkylreste, gegebenenfalls mit einer oder mehreren Unsättigungen, mit 1 bis 12 Kohlenstoffatomen inbetracht. Insbesondere sind alle den für $R^1$ und $R^3$ genannten Acylgruppen entsprechenden Alkylreste zu nennen. Besonders bevorzugt ist die Methylgruppe.

Für $R^2$ ist ein Wasserstoffatom der bevorzugte Rest.

Für das Fragment

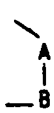

kann entweder eine C15-C16-Doppelbindung stehen oder aber eine C15-C16-Alkylengruppe

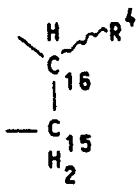
.

die in der 16-Position mit einem α- oder ß-ständigen Alkyl- oder einem gegebenenfalls mehrfach ungesättigten Alk-1,2-enylrest mit 1 bis 8 bzw. 2 bis 8 Kohlenstoffatomen substituiert ist. Es sind dies vor allem die Methyl-, Ethyl-,, Propyl-, Isopropyl-, Propenyl-, Butyl-, t-Butyl-, Butadienyl-, Cyclopentylmethyl- und Cyclopentylmethylen- sowie die Hexylgruppe, wovon die Methylgruppe als besonders bevorzugte zu nennen ist.

Als Substituent $R^4$ kommt schließlich auch eine geradkettige Alk-1,2-inyl-Gruppe mit 2 bis 8 Kohlenstoffatomen infrage. Die Ethinyl-und Propionylgruppe sind bevorzugt.

Als den Verbindungen der allgemeinen Formel I am nächsten kommende Verbindungen sind in J. Chem. Soc., Chem. Commun., 1986, 451-453, 14α,17α-Etheno- und in der internationalen Patentanmeldung PCT/DE87/00361 14α,17α-Etheno- und 14α,17α-Ethano-überbrückte Steroide mit den gleichen Substituenten in 3- und 17-Stellung wie in den erfindungsgemäßen Verbindungen beschrieben; in 13-Position weisen die bekannten Verbindungen eine Methylgruppe auf. Während die hier vorliegenden Verbindungen der allgemeinen Formel I die C15-C16-Doppelbindung oder den Alkylrest am C16-Atom im oberen Ring D des Steroidgerüstes aufweisen, befindet sich die C15-C16-Doppelbindung oder der C16-Alkylsubstituent im/am unteren D-Ring des Steroidgerüstes bei gleicher Anordnung (jeweils ß) des 17-OR-Substituenten (R = H, Acyl). Mit anderen Worten: bei den vorliegenden Verbindungen der Formel I handelt es sich um 14α,17α-Ethano-estratetraene (A) bzw. 14α,17α-Ethano-16-alkyl-estratriene (B), während es sich bei den bereits bekannten Verbindungen um 14α,17α-Etheno-estratriene (C) bzw. um 14α,17α-Alkylethano-estratriene (D) handelt:

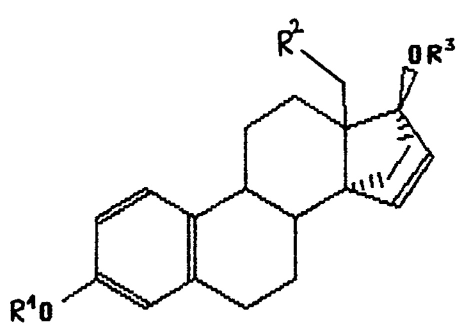

(A)

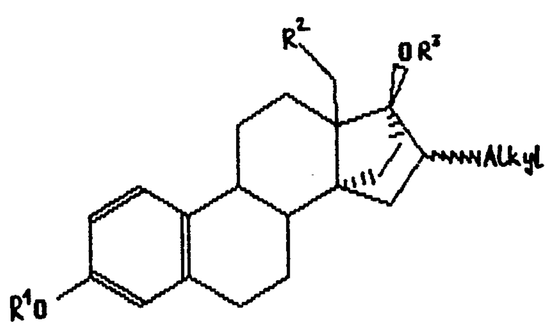

(B)

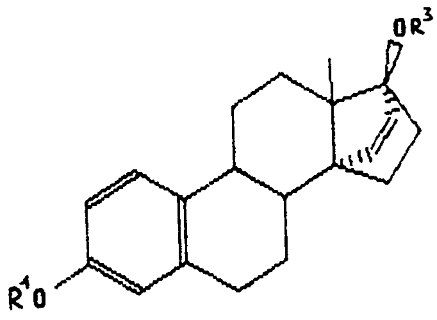

(C)

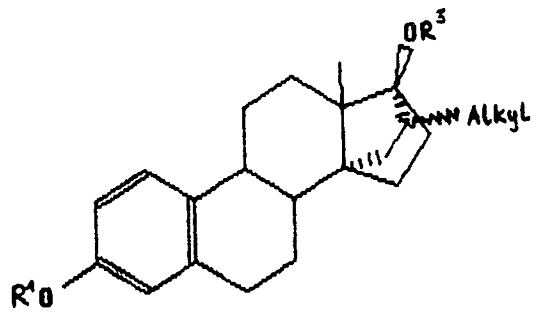

(D)

Aus der PCT/DE87/00361 geht weiterhin hervor, daß sich die 14α,17α-Etheno-estratriene vom Typ C zu den ebenfalls stark östrogen-wirksamen, entsprechenden 15,16-Dihydroverbindungen hydrieren lassen.

Ebenso wie die zum Stand der Technik gehörenden Verbindungen zeichnen sich die erfindungsgemäßen Verbindungen durch eine außerordentlich starke östrogene Wirksamkeit aus. Im Allen-Doisy-Test sind sie nach subcutaner Applikation mindestens ebenso stark östrogen wirksam wie Ethinylestradiol und von vergleichbarer Wirksamkeit wie die bereits bekannten Verbindungen vom Typ (C).

Im Allen-Doisy-Test nimmt man eine Bewertung von Vaginalabstrichen bei ovariektomierten Ratten an den Tagen 3, 4, 5 und 8 nach der einmaligen Applikation der Prüfsubstanz vor. Folgende Zyklusstadien werden unterschieden:

1 = Diöstrus (Leukozyten und kernhaltige Epithelzellen),
2 = Proöstrus (kernhaltige Epithelzellen),
3 = Östrus (kernlose Hornschollen),
4 = Metöstrus (kernlose Hornschollen, Leukozyten, Epithelzellen).

Östrogen wirksame Substanzen führen nach oraler oder subcutaner Applikation zur Proliferation des Vaginalepithels und zur Verhornung der oberflächlichen Zell-Lagen. Als Schwellenwert wird diejenige Menge eines Estrogens angesehen, bei der 50 % der Tiere Stadium 3 erreichen.

Die Ergebnisse des Allen-Doisy-Tests mit 14α,17α-Ethano-1,3,5(10),15estratetraen-3,17ß-diol als Testverbindung sowie 14α,17α-Ethano-1,3,5(10)estratrien-3,17ß-diol und Estradiol als Vergleichsverbindungen sind in Tabelle 1 zusammengefaßt. Die Uterigewichte werden nach Autopsie von denjenigen Tieren bestimmt, an denen zuvor die Vaginalabstriche durchgeführt wurden.

Tabelle 1

Vaginalabstrichtest nach Allen u. Doisy (A 1.1. modifiziert.) an Ratten bei s.c. Applikation Injektion 1X an d1 ; Autopsie d5 ; n = 6 / Gruppe ; Kontrolle n = 12

| Behandlung | | Differenz KG | | Uterusgew.(mg) | | | | Uterusgew.(mg)/100g Kg | | | | Abstriche n = pos./n = Beh. |
|---|---|---|---|---|---|---|---|---|---|---|---|---|
| | | d5 — d1 (g) | | (feucht) | | (trocken) | | (feucht) | | (trocken) | | |
| | | Mean | SD | Mean | SD | Mean | SD | Mean | SD | Mean | SD | |
| 14α,17α-Ethano-1,3,5(10),15-estratetraen-3,17β-diol | / 3,00 ug | -0.33 | 3.56 | 713.92 | 368.43 | 135.83 | 54.62 | 335.57 | 156.29 | 64.61 | 23.10 | 6 / 6 |
| | / 1,00 ug | 6.50 | 2.59 | 415.62 | 250.80 | 77.23 | 37.73 | 199.21 | 129.50 | 36.92 | 19.64 | 6 / 6 |
| | / 0,30 ug | 11.50 | 2.26 | 238.45 | 25.65 | 44.93 | 3.84 | 110.19 | 14.62 | 20.74 | 2.21 | 6 / 6 |
| | / 0,10 ug | 16.50 | 6.98 | 156.62 | 10.85 | 29.23 | 2.76 | 70.72 | 6.58 | 13.22 | 1.70 | 6 / 6 |
| | / 0,03 ug | 18.17 | 4.75 | 128.07 | 17.22 | 25.38 | 5.28 | 58.89 | 6.68 | 11.65 | 2.17 | 0 / 6 |
| 14α,17α-Ethano-1,3,5(10)-estratrien-3,17β-diol | / 3,00 ug | 0.67 | 6.86 | 576.37 | 248.15 | 92.98 | 21.53 | 276.18 | 113.26 | 44.81 | 10.05 | 6 / 6 |
| | / 1,00 ug | 2.83 | 4.83 | 327.72 | 37.87 | 66.87 | 13.52 | 158.32 | 23.83 | 32.38 | 7.77 | 6 / 6 |
| | / 0,30 ug | 11.33 | 3.56 | 299.50 | 71.34 | 45.72 | 6.04 | 135.06 | 34.94 | 20.50 | 2.28 | 6 / 6 |
| | / 0,10 ug | 10.83 | 2.79 | 191.80 | 105.71 | 28.18 | 5.62 | 89.92 | 51.58 | 13.14 | 2.58 | 5 / 6 |
| | / 0,03 ug | 17.33 | 3.72 | 134.80 | 41.83 | 24.18 | 3.13 | 60.90 | 22.05 | 10.83 | 1.51 | 2 / 6 |
| Estradiol | / 3,00 ug | 7.67 | 5.28 | 367.28 | 108.65 | 71.65 | 14.70 | 170.30 | 49.06 | 33.38 | 7.18 | 6 / 5 |
| | / 1,00 ug | 5.17 | 2.23 | 234.77 | 21.88 | 59.55 | 19.30 | 111.65 | 9.93 | 28.42 | 9.49 | 6 / 6 |
| | / 0,30 ug | 12.00 | 2.10 | 192.10 | 40.03 | 42.18 | 17.18 | 89.32 | 18.98 | 19.57 | 8.01 | 6 / 6 |
| | / 0,10 ug | 14.33 | 3.56 | 137.05 | 17.94 | 25.07 | 3.50 | 61.85 | 9.30 | 11.27 | 1.44 | 6 / 6 |
| | / 0,03 ug | 20.17 | 4.75 | 123.17 | 13.29 | 23.60 | 4.98 | 54.85 | 5.67 | 10.52 | 2.32 | 0 / 6 |
| Kontrolle | | 21.08 | 4.25 | 123.72 | 20.19 | 27.58 | 6.09 | 54.20 | 11.27 | 12.09 | 3.11 | 0 / 12 |

( M ± SD—p <0,05 vs. Kontrolle, LSD - Test )

Als weitere Verbindung wurde 14α,17α-Ethano-1,3,5(10)-estratrien-16α-ethinyl-3,17β-diol getestet; bei einer Dosis von 0,3 µg/d s.c. weisen alle 6 untersuchten Tiere positive Abstriche auf.

Es ist überraschend, daß die erfindungsgemäßen Verbindungen, die keine 17α-Ethinylgruppe enthalten, mindestens ebenso wirksam sind wie Ethinylestradiol. Ethinylestradiol ist nach wie vor das bei oraler Behandlung am häufigsten eingesetzte Östrogen.

Gegenüber Ethinylestradiol bieten die erfindungsgemäßen Verbindungen den Vorteil, daß sie keine 17ß-Hydroxygruppe aufweisen, die zu unerwünschten metabolischen Reaktionen Anlaß geben könnte.

Wie noch auszuführen sein wird, sind sie außerdem nach einem wesentlich einfacheren Verfahren als die strukturell nahestehenden Verbindungen vom Typ (C) herstellbar.

Die Erfindung betrifft somit auch Verbindungen der allgemeinen Formel I zur Verwendung bei der Behandlung von Östrogenmangelerscheinungen und zur Fertilitätskontrolle bei der Frau.

Die erfindungsgemäßen Verbindungen können in der gleichen Weise wie Ethinylestradiol formuliert und eingesetzt werden. Sie werden mit den in der galenischen Pharmazie üblichen Zusätzen, Trägersubstanzen und/oder Geschmackskorrigentien nach an sich bekannten Methoden zu den üblichen Arzneimittelformen verarbeitet. Für die orale Applikation kommen insbesondere Tabletten, Dragees, Kapseln, Pillen, Suspensionen oder Lösungen infrage. Für die parenterale Applikation kommen insbesondere ölige Lösungen, wie zum Beispiel Sesamöl- oder Rizinusöllösungen, infrage, die gegebenenfalls zusätzlich noch ein Verdünnungsmittel, wie zum Beispiel Benzylbenzoat oder Benzylalkohol, enthalten können.

Die Wirkstoffkonzentration in den pharmazeutischen Zusammensetzungen ist abhängig von der Applikationsform und dem Anwendungsgebiet. So können zum Beispiel Kapseln oder Tabletten zur Behandlung von Östrogenmangelerscheinungen 0,001 bis 0,05 mg Wirkstoff, ölige Lösungen zur intramuskulären Injektion pro 1 ml etwa 0,01 bis 0,1 mg Wirkstoff und Vaginalsalben etwa 0,1 bis 10 mg pro 100 ml Salbe enthalten. Zur Kontrazeption bei der Frau können die erfindungsgemäßen Östrogene in Kombination mit Gestagenen angewandt werden. Tabletten oder Dragees zur täglichen Einnahme einer Tablette oder eines Dragees sollen vorzugsweise 0,003 bis 0,05 mg des erfindungsgemäßen Östrogens und 0,05 bis 0,5 mg eines Gestagens enthalten.

Die erfindungsgemäßen Verbindungen können bei Östrogenmangelerscheinungen der Frau, wie zum Beispiel Amenorrhoe, Dysmenorrhoe, Sterilität, Frigidität, Endometritis, Kolpitis, und klimakterischen Beschwerden verwendet werden. Ferner können die Verbindungen als östrogene Komponente in Kombinationspräparaten mit Gestagenen zur Fertilitätskontrolle bei der Frau eingesetzt werden.

Die neuen Verbindungen der allgemeinen Formel I

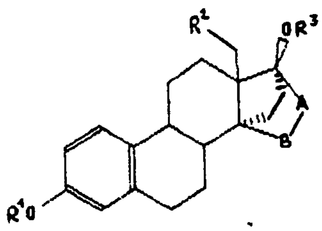

(I),

worin $R^1$, $R^2$, $R^3$ und

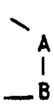

die bereits angegebene Bedeutung haben, werden erfindungsgemäß so hergestellt, daß

a) wenn

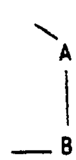

für

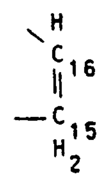

steht,
eine Verbindung der allgemeinen Formel IIIa

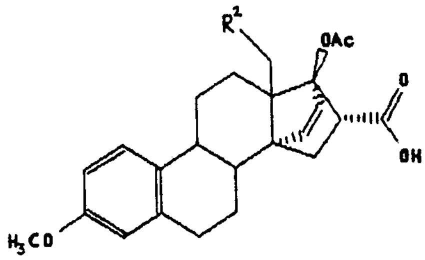

(IIIa),

worin
$R^2$ die in Formel I angegebene Bedeutung hat
zunächst hydriert und dann
zu einer Verbindung der allgemeinen Formel II′a

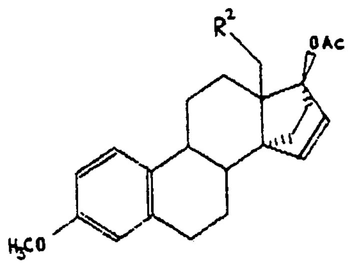

(II′a)

decarboxyliert und oxidiert wird,
b) oder wenn

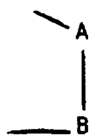

für

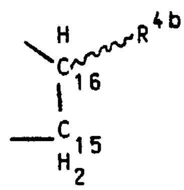

mit $R^{4b}$ in der Bedeutung einer Methylgruppe steht,
eine Verbindung der allgemeinen Formel IIIb

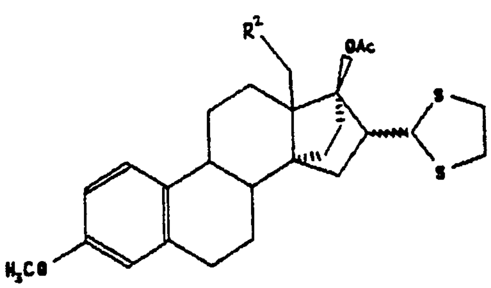

(IIIb),

worin $R^2$ die bereits angegebene Bedeutung hat,
zu einer Verbindung der allgemeinen Formel II′b

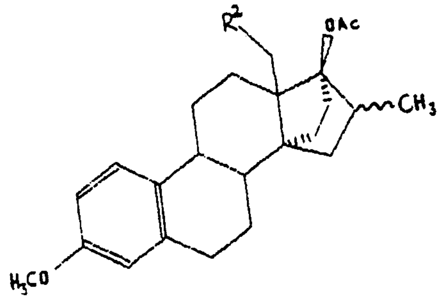

(II'b)

reduziert wird,

c) oder wenn

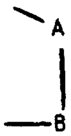

für

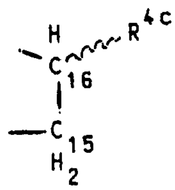

mit $R^{4c}$ in der Bedeutung eines Alkyl- oder eines gegebenenfalls mehrere Doppelbindungen aufweisenden α,ß-Alkenylrestes, beide mit 2 bis 8 Kohlenstoffatomen, steht, eine Verbindung der allgemeinen Formel IIIc

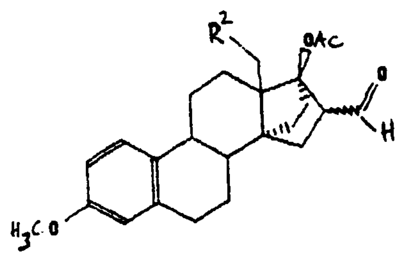

(IIIc),

worin $R^2$ die bereits angegebene Bedeutung hat, mit einem entsprechenden Wittig-Reagenz zu einer Verbindung der allgemeinen Formel II″C

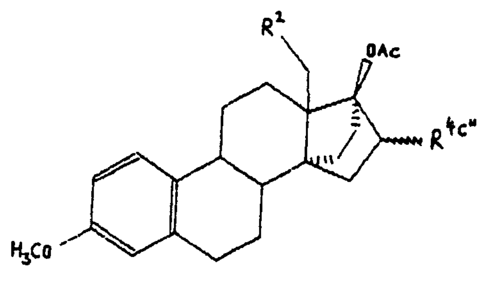

(II″c),

worin $R^{4c''}$ einen gegebenenfalls mehrere Doppelbindungen aufweisenden α,ß-Alkenylrest mit 2 - 8 Kohlenstoffatomen darstellt, umgesetzt wird, die Verbindung der allgemeinen Formel II″c gegebenenfalls zu einer Verbindung der allgemeinen Formel II′c

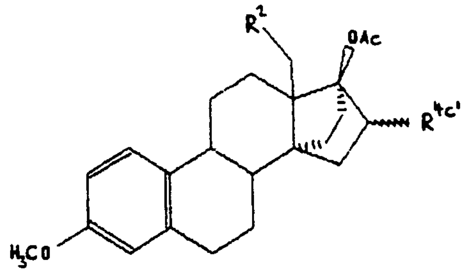

(II'c),

worin $R^{4c'}$ einen Alkylrest mit 2 bis 8 Kohlenstoffatomen darstellt, hydriert wird

d) oder wenn

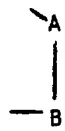

für

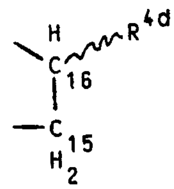

mit $R^{4d}$ in der Bedeutung eines α,β-Alkinylrestes mit 2 bis 8 Kohlenstoffatomen steht, eine Verbindung der allgemeinen Formel III c

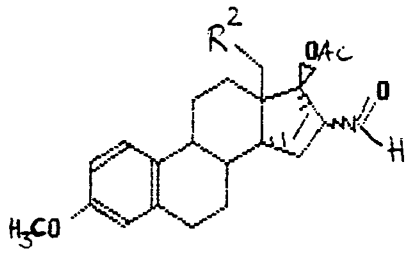

(IIIc)

in einer modifizierten Wittig-Reaktion mit einem Gemisch aus Triphenylphosan und Tetrahalogenmethan $CHal_4$, wobei Hal Brom und Jod, vorzugweise Brom, sein kann, zu einer Verbindung der allgemeinen Formel II''d

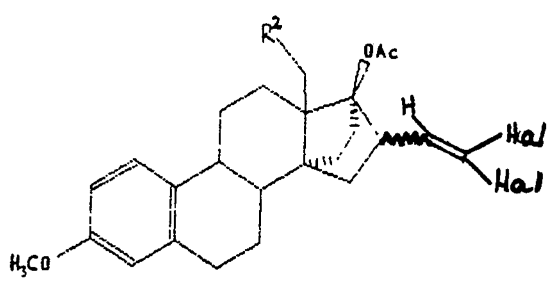

(II''d)

umgesetzt wird und anschließend in einer Verbindung einer der allgemeinen Formeln II'a, II'b, II''c, II'c oder II''d gewünschtenfalls den 3-Methylether spaltet und

falls Verfahrensvariante d) vorliegt, nach Überführung der Verbindung der allgemeinen Formel II''d oder der entsprechenden 3-Hydroxyverbindung durch Abspaltung von HHal mit einer Base und Halogen-/Wasserstoffaustausch und gegebenenfalls nach Kupplung der entstandenen H-Ethinylverbindung mit einem 1 bis 6 Kohlenstoffatome aufweisenden linearen Alkylierungsreagenz in eine Verbindung der allgemeinen Formel II'd

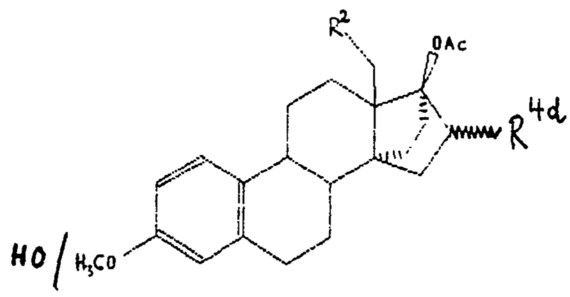

(II'd),

worin $R^{4d}$ die bereits angegebene Bedeutung hat und anschließend gewünschtenfalls die freigesetzte 3-Hydroxygruppe wieder verethert und/oder die 17-Acetoxygruppe verseift, gegebenenfalls die 3-und 17-Hydroxygruppen wieder verestert und gegebenenfalls eine so erhaltene 3,17-Diacyloxyverbindung selektiv zur 3-Hydroxy-17-acyloxyverbindung verseift.

Je nach dem, welches Zwischen- bzw. Endprodukt der allgemeinen Formeln II′a, II′b, II″c, II′c oder II″d (die Verbindungen dieser Formeln können auch bereits Endprodukte der allgemeinen Formel I sein, wenn $R^1$ eine Methyl- und $R^3$ eine Acetylgruppe sein soll) gewünscht ist, sind erfindungsgemäß unterschiedliche Vorgehensweisen erforderlich.

a) Der D-Ring soll eine C15-C16-Doppelbindung aufweisen:
ausgehend von einem 16α-Carbaldehyd der allgemeinen Formel IVa

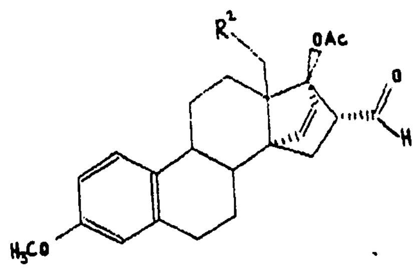

(IVa),

worin $R^2$ für ein H-Atom oder eine Methylgruppe steht, wird durch Oxidation der Carbonylgruppe, beispielsweise mit Jones-Reagenz (Chromtrioxid/Schwefelsäure) die entsprechende Carboxylverbindung IIIa

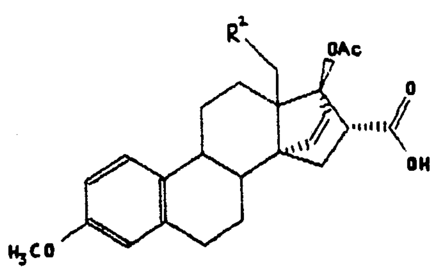

(IIIa)

hergestellt. Durch Hydrierung der 14α,17α-Ethenobrücke, Decarboxylierung und Oxidation (Einführung der C15-C16-Doppelbindung) wird IIIa in eine Verbindung der allgemeinen Formel II′a

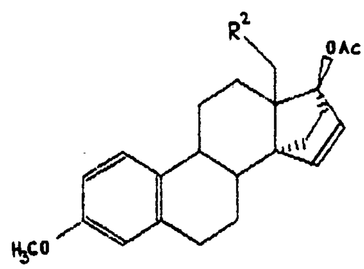

(II'a)

überführt. Die Decarboxylierung und Oxidation wird durch Erhitzen in einem Lösungsmittel unter Einwirkung mindestens eines Oxidationsmittels, beispielsweise mit Bleitetraacetat und Kupfer(II)-acetathydrat in Benzol oder Toluol, bewerkstelligt.

b) Der D-Ring soll eine C16-α- oder ß-Methylgruppe besitzen:
es wird eine Verbindung der allgemeinen Formel IIIc

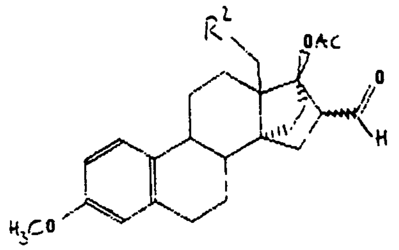

(IIIc),

worin $R^2$ für ein H-Atom oder eine Methylgruppe steht und die Aldehydfunktion α- oder ß-ständig sein kann, durch Umsetzung mit 1,2-Dimercaptoethan in ihr Ethylen-1,2-dithioketal-Derivat IIIb

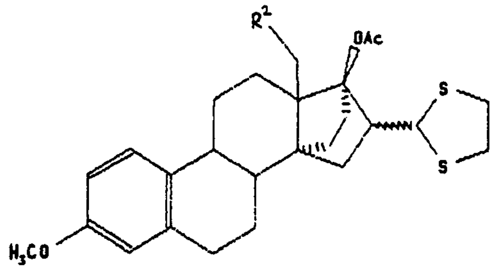

(IIIb)

umgewandelt; diese Verbindung der Formel IIIb wird anschließend in einem protischen Lösungsmittel zu einer Verbindung der allgemeinen Formel II'b

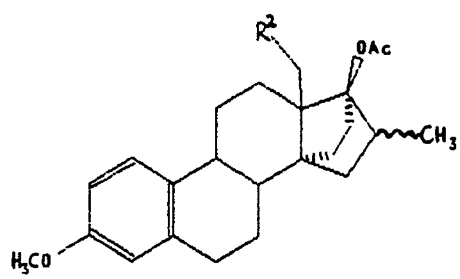

(II'b)

reduziert, beispielsweise mit Raney-Nickel in Alkohol.

c) Der D-Ring soll einen C16-α- oder ß-Alkyl- oder einen gegebenenfalls mehrere Doppelbindungen aufweisenden C-16α- oder ß-Alkenylrest mit 2 bis 8 Kohlenstoffatomen tragen:
ebenfalls ausgehend von einem Aldehyd der Formel IIIc wird durch eine C-C-Verknüpfungsreaktion die 16α- oder 16ß-Alkenyl- bzw. Alkylseitenkette aufgebaut, beispielsweise„durch Wittig-Reaktion von IIIc mit einem geeigneten Wittig-Reagenz, d.h. mit einem um ein C-Atom als $R^{4c}$ ärmeren Wittig-Reagenz.
Die in der C16-Seitenkette durch die Wittig-Reaktion entstandene Doppelbindung (sowie gegebenenfalls vorhandene weitere Doppelbindungen) kann (können) gewünschtenfalls durch Hydrieren wieder beseitigt werden.

Durch die unter c) beschriebene Variante kommt man also sowohl zu Verbindungen der allgemeinen Formel II''c

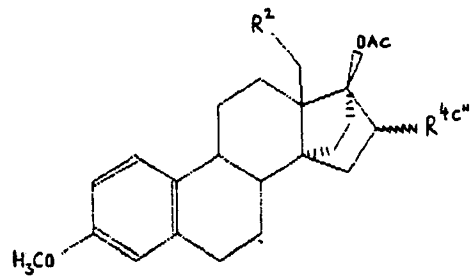

(II"c),

worin $R^{4c''}$ einen mindestens einfach-ungesättigten Alkenylrest mit 2 bis 8 Kohlenstoffatomen darstellt als auch zu Verbindungen der allgemeinen Formel II'c

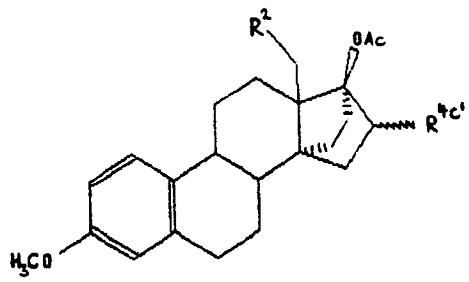

(II'c),

worin nunmehr in $R^{4c'}$ die in $R^{4c''}$ vorhanden gewesenen Doppelbindungen hydriert sind.

d) Der D-Ring soll einen geradkettigen C16α- oder C16ß-Alk-1,2-inylrest mit 2 bis 8 Kohlenstoffatomen als Substituent aufweisen:

ein Aldehyd der Formel IIIc als Ausgangsprodukt wird in einer modifizierten Wittig-Reaktion mit einem Gemisch aus Triphenylphosphin und Tetrabrom- oder Tetrajodmethan umgesetzt. Als reaktive Spezies fungiert dabei Triphenyl-dibrom (bzw. dijod) methylen-phosphoran. Aus dem entstandenen Wittig-Reaktionsprodukt der allgemeinen Formel II"d wird dann mit einer Base wie beispielsweise n-Butyllithium Bromwasserstoff oder Jodwasserstoff eliminiert. Bei der wässrigen Aufarbeitung des nach der Eliminierung erhaltenen Reaktionsproduktes erhält man die entsprechende 16α- oder 16ß-Ethinylverbindung; wird nach der Halogenwasserstoffabspaltung ein n-Alkylierungsreagenz mit 1 bis 6 Kohlenstoffatomen im Alkylrest zugesetzt, gelangt man zu Verbindungen der allgemeinen Formel II'd, in denen $R^4$ ein α- oder ß-ständiger Alk-1,2-inylrest mit 3 bis 8 Kohlenstoffatomen ist. So führt beispielsweise die Aufarbeitung in Gegenwart von Methyljodid zur entsprechenden 16-Prop-1,2-inylverbindung.

Die vorliegende Erfindung bezieht sich auch auf die Verbindungen der Formel IV

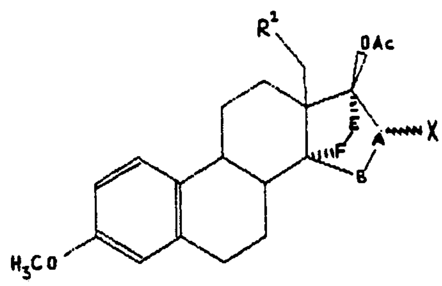

(IV),

worin

$R^2$ für ein Wasserstoffatom oder eine Methylgruppe steht und

entweder

X eine α-ständige Carbaldehydgruppe

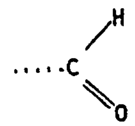

und

A-B eine C-C-Einfachbindung sowie E-F eine C-C-Doppel- oder C-C-Einfachbindung

oder

X eine ß-ständige Carbaldehydgruppe

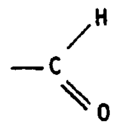

und

A-B und E-F zwei C-C-Doppel- oder zwei C-C-Einfachbindungen

bedeuten,

da diese Verbindungen wertvolle und vielfältig einsetzbare Zwischenprodukte darstellen wegen der chemisch vielseitigen Ketogruppe.

Die Herstellung der Verbindungen der allgemeinen Formel IV erfolgt ausgehend von 3-Methoxy-1,3,5-(10),14,16-estrapentaen-17-ylacetat ($R^2 = H$)

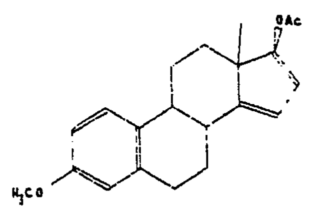

[G.H. Rasmusson et al., Steroids 22, 107, (1973)] bzw. ausgehend von 3-Methoxy-1,3;5(10),14,16-estrapentaen-18-methyl-17-yl-acetat ($R^2 = CH_3$) durch Umsetzung mit einem in Nachbarstellung zur Ketogruppe ungesättigten Aldehyd. Eine Vorschrift zur Darstellung der letztgenannten Verbindung findet sich in Beispiel 16. Beispielsweise liefert Umsetzung der ersten Verbindung ($R^2 = H$) mit Acrolein in der Kälte und in Gegenwart einer Lewis-Säure das [4+2]-Cycloaddukt

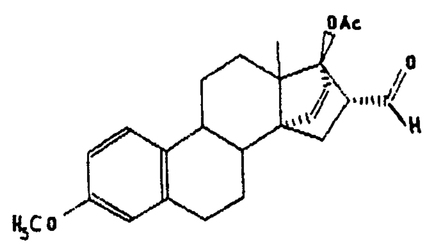

mit α-ständiger Aldehydfunktion am C16-Atom

während sich mit Propinal in der Hitze das [4+2]-Cycloaddukt

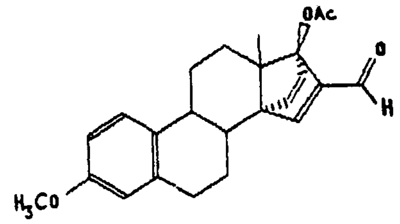

mit ß-ständiger Aldehydfunktion am C16-Atom bildet.

Beide Cycloaddukte lassen sich katalytisch, beispielsweise mit Palladium-Aktivkohle, hydrieren (auch wenn $R^2 = CH_3$).

Zeichnen sich also die erfindungsgemäßen Verbindungen der allgemeinen Formel I insbesondere durch ihre hohe östrogene Wirksamkeit aus, so können sie außerdem nach einem relativ einfachen Verfahren und bequem über die Verbindungen der allgemeinen Formel IVa bzw. IIIc hergestellt werden.

Die bekannten 14α,17α-Etheno-1,3,5(10)-estratriene (C) werden nämlich bisher durch Reduktion der entsprechenden 16-Phenylsulfone der Formel

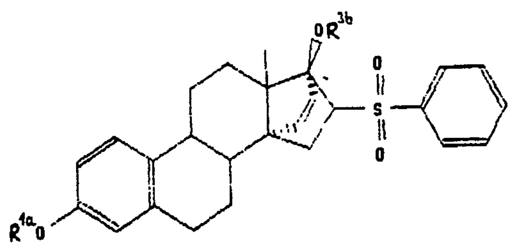

worin

$R^{1a}$ = $R^1$ und $R^{3a}$ = $R^3$ oder Vorläufer von $R^1$ bzw. $R^3$

bedeuten,

mit einem Überschuß von 6 %igem Natriumamalgam hergestellt (J. Chem. Soc., Chem. Commun. 1986, 451-453). Beispielsweise werden zur reduktiven Eliminierung der Phenylsulfongruppe aus 2 g 14α,17α-Etheno-3-methoxy-16α-phenylsulfonyl-1,3,5(10)-estratrien-17ß-ol-acetat auf herkömmliche Weise 31,5 g 6 %iges Natriumamalgam benötigt, die fast 30 g Quecksilber enthalten. Aus Gründen der Wirtschaftlichkeit und wegen der extremen Toxizität des Quecksilbers ist eine solche Synthese nicht in einen vernünftigen technischen Maßstab übertragbar, da beispielsweise um 100 kg des oben genannten Sulfons zu reduzieren, 1,5 Tonnen Quecksilber erforderlich wären. Demgegenüber vermeidet das vorliegende Verfahren diese Nachteile und Beeinträchtigungen, die bei Umgang mit Quecksilber auftreten.

Außerdem lassen sich die erfindungsgemäßen Verbindungen der allgemeinen Formel I, in denen

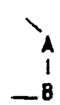

für eine C15-C16-Doppelbindung steht, wie in Beispiel 15 gezeigt ist, hydrieren, wodurch man zu identischen 15,16-Dihydroverbindungen gelangt wie durch Hydrierung der bekannten 14α,17α-Etheno-estratriene vom Typ C.

Die vorliegende Erfindung wird anhand der nachfolgenden Beispiele näher erläutert.

**Beispiel 1**

17ß-Acetoxy-14α,17α-etheno-3-methoxy-1,3,5(10)-estratrien-16α-carbaldehyd (2)

10,0 g (30,8 mMol) 3-Methoxy-1,3,5(10),14,16-estra-pentaen-17-ylacetat (1) [G. M. Rasmusson et al. Steroids 22, 107 (1973)] und 4,17 ml (62,7 mMol) Acrolein in 124 ml Toluol wurden bei Eiskühlung mit 0,19

ml (1,6 mMol) Bortrifluorid-Etherat in 11,4 ml Toluol tropfenweise versetzt. Die Reaktionsmischung wurde 16 Stunden bei Raumtemperatur gerührt und danach auf Eis/Wasser gegeben. Anschließend wurde mit Essigester extrahiert und die organische Phase nach dem Waschen mit Natriumhydrogencarbonat-Lösung und Wasser über Natriumsulfat getrocknet. Filtration und Verdampfung des Lösungsmittels ergaben 9,9 g festen Rückstand. Durch Chromatographie an Kieselgel mit Essigester/Hexan (1:2 → 1:1) wurden 8,55 g (2) vom Schmelzpunkt 183 - 185 °C erhalten.
$[\alpha]_D^{20}$ +102,5° (C 0,120, $CHCl_3$)

**Beispiel 2**

17ß-Acetoxy-14α,17α-etheno-3-methoxy-1,3,5(10)-estratrien-16α-carbonsäure (3)

8,0 g (21,0 mMol) 2 in 180 ml Aceton wurden mit 11,0 ml (29,3 mMol) Jones-Reagenz (2,67 g Chromtrioxid in 7,7 ml Wasser und 2,3 ml konz. Schwefelsäure) bei 0 °C tropfenweise versetzt. Die Reaktionsmischung wurde noch 3 1/2 Stunden bei Eiskühlung und 1 Stunde bei Raumtemperatur gerührt und danach auf Eis/Wasser gegeben. Anschließend wurde mit Essigester extrahiert, die organische Phase mit Wasser gewaschen und über Natriumsulfat getrocknet. Filtration und Entfernung des Lösungsmittels lieferten 8,03 g (3) vom Schmelzpunkt 194 - 198 °C.

**Beispiel 3**

17ß-Acetoxy-14α,17α-ethano-3-methoxy-1,3,5(10)-estratrien-16α-carbonsäure (4)

8,0 g (3) wurden in 672 ml Äthanol in Gegenwart von 1,76 g Pd-C (10 %) bei Normaldruck hydriert. Nach Filtration und Verdampfung des Lösungsmittels wurden 7,5 g (4) als Rohprodukt vom Schmelzpunkt 224 - 227 °C erhalten.

**Beispiel 4**

14α,17α-Ethano-3-methoxy-1,3,5(10),15-estratetraen-17ß-ol-acetat (5)

7,45 g (18,7 mMol) (4) und 0,65 g (3,26 mMol) Kupfer-II-acetat-hydrat wurden in 1,5 ml Pyridin und 137 ml Benzol zunächst 30 Minuten bei Raumtemperatur und nach Zugabe von 13,5 g (30,4 mMol) Bleitetraacetat bei 90 °C 4 Stunden in einer Stickstoffatmosphäre unter Lichtausschluß gerührt. Die Reaktionsmischung wurde durch Kieselgel filtriert, mit 2 Liter Essigester/Hexan (1:1) nachgewaschen und das Filtrat eingeengt. Durch Chromatographie des öligen Rückstandes an Kieselgel mit Essigester/Hexan (1:1) wurden 1,27 g (5) als hellgelbes Öl erhalten, zusätzlich 1,16 g verunreinigt.

**Beispiel 5**

14α,17α-Ethano-1,3,5(10),15-estratetraen-3,17ß-diol (6)

1,18 g (3,35 mMol) (5) in 354 ml Toluol wurden mit 88,5 ml (106,2 mMol) Diisobutylaluminiumhydrid (1,2 molar in Toluol) 14 Stunden in einer Stickstoffatmosphäre unter Rückfluß gehalten. Die abgekühlte Reaktionsmischung wurde auf Eis/verd. Essigsäure gegeben. Danach wurde mit Essigester extrahiert und die organische Phase nach dem Waschen mit Natriumhydrogencarbonat-Lösung und Wasser über Natriumsulfat getrocknet. Nach Filtration und Verdampfung des Lösungsmittels wurden 1,3 g Feststoff erhalten, die nach Chromatographie an Kieselgel mit Hexan → Hexan/Essigester (1:1) 540 mg (6) vom Schmelzpunkt 200 °C ergaben.
$[\alpha]_D^{20}$ -11,4° (C 0,105; $CHCl_3$).

**Beispiel 6**

17ß-Acetoxy-14α,17α-ethano-3-methoxy-1,3,5(10)-estratrien-16α-carbaldehyd (7)

2,0 g (2) in 250 ml Essigester wurden mit 0,5 g Pd-C (10 %) bei Normaldruck hydriert. Nach Entfernung des Katalysators und Verdampfung des Lösungsmittels wurden 1,99 g (7) vom Schmelzpunkt 151 - 152 °C erhalten.

**Beispiel 7**

16α-(1,3-Dithiolan-2-yl)-14α,17α-ethano-3-methoxy-1,3,5(10)-estratrien-17ß-ol-acetat (8)

6,51 g (17,0 mMol) (7) und 2,26 ml (26,9 mMol) 1,2-Dimercaptoäthan wurden in 15 ml Methylenchlorid gelöst und bei 0 °C mit 0,6 ml (4,76 mMol) Bortrifluorid-Etherat in 5,9 ml Methylenchlorid bei 0 °C tropfenweise versetzt. Die Reaktionsmischung wurde noch 3 Stunden bei Raumtemperatur gerührt, mit Essigester verdünnt und mit 5 %iger Natronlauge und Wasser gewaschen. Trocknung der organischen Phase über Natriumsulfat, Filtration und Verdampfung des Lösungsmittels lieferten 7,62 g Rohprodukt von (8), das zur Raney-Nickel-Entschwefelung eingesetzt wird.

**Beispiel 8**

14α,17α-Ethano-3-methoxy-1,3,5(10)-estratrien-16α-methyl-17ß-ol-acetat (9)

7,52 g von (8) wurden in 400 ml Äthanol zusammen mit ca. 15 g Raney-Nickel in einer Stickstoffatmosphäre unter Rückfluß gehalten. Nach Filtration und Entfernung des Lösungsmittels wurden 5,60 g (9) vom Schmelzpunkt 115 °C erhalten.

**Beispiel 9**

14α,17α-Ethano-16α-methyl-1,3,5(10)-estratrien-3,17ß-diol (10)

2,0 g (5,42 mMol) (9) wurden mit 76,0 ml (91,4 mMol) Diisobutylaluminiumhydrid (1,2 molar in Toluol) 5 1/2 Stunden unter Stickstoff bei Rückfluß gehalten. Die abgekühlte Reaktionsmischung wurde mit Toluol verdünnt unt auf Eis/verdünnte Essigsäure gegeben. Nach Zusatz von Kochsalz wurde mit Essigester extrahiert und die organische Phase mit Natriumhydrogencarbonat-Lösung und Wasser gewaschen. Nach Trocknung über Natriumsulfat; Filtration und nach Abziehen des Lösungsmittels wurden 1,71 g Feststoff erhalten. Umkristallisation in Aceton/Hexan ergaben 610 mg (10) vom Schmelzpunkt 264 - 265 °C.
$[\alpha]_D^{20}$ +30,4° (C 0,105; $CHCl_3$).

**Beispiel 10**

17ß-Acetoxy-14α,17α-etheno-3-methoxy-1,3,5(10),15-estratetraen-16-carbaldehyd (11)

10,0 g (30,8 mMol) (1) und 28,21 g einer Mischung aus Propinal, 2-Butanon und Diethylether [hergestellt nach M.G. Veliev et al. Synthesis 461 (1980); Ansatz: 36,5 g (0,65 Mol) Propargylalkohol; das Produkt enthält nach vorschriftsgemäßer Aufarbeitung noch 2-Butanon und Diethylether] in 114 ml Toluol wurden 10 Stunden unter Rückfluß gehalten. Die abgekühlte Reaktionsmischung wurde eingeengt (15,57 g kristallisierendes Öl) und in Essigester/Hexan umkristallisiert. Es wurden 7,84 g (11) vom Schmelzpunkt 155,5 °C erhalten.

$[\alpha]_D^{20}$ -52,3 (C 0,105; $CHCl_3$).

**Beispiel 11**

17ß-Acetoxy-14α,17α-ethano-3-methoxy-1,3,5(10)-estratrien-16ß-carbaldehyd (12)

2,0 g (11) wurden mit 400 mg Pd-C (10 %) in 40 ml Essigester bei Normaldruck hydriert. Nach Filtration und nach Abziehen des Lösungsmittels verblieben 2,06 g Feststoff. Das Rohrprodukt - nach $^1$H-NMR bestehend aus 85 % 16β- und 15 % 16α-Carbaldehyd - wird zur Thioketalisierung eingesetzt.

**Beispiel 12**

16ß-(1,3-Dithiolan-2-yl)-14α,17α-ethano-3-methoxy-1,3,5(10)-estratrien-17ß-ol-acetat (13)

3,0 g (7,894 mMol) (12) und 1,04 ml (12,4 mMol) 1,2-Dimercaptoäthan wurden in 7 ml Methylenchlorid gelöst und bei 0 °C mit 0,28 ml (2,20 mMol) Bortrifluorid-Etherat in 2,7 ml Methylenchlorid tropfenweise versetzt. Die Reaktionsmischung wurde 20 Minuten bei 0 °C und 3 Stunden bei Raumtemperatur gerührt. Nach Verdünnung mit Essigester wurde die Reaktionsmischung mit 5 %iger Natronlauge und Wasser gewaschen. Die organische Phase wurde über Natriumsulfat getrocknet, filtriert und zur Trockne eingeengt. Man erhielt 3,06 g (13) als Rohprodukt, das zur Raney-Nickel-Entschwefelung eingesetzt wird.

**Beispiel 13**

14α,17α-Ethano-3-methoxy-1,3,5(10)-estratrien-16ß-methyl-17ß-ol-acetat (14)

3,0 g (13) wurden zusammen mit ca. 7,5 g Raney-Nickel in 200 ml Äthanol unter Schutzgas bei Rückfluß gehalten. Nach Abkühlung, Filtration und Verdampfung des Lösungsmittels wurden 2,40 g farbloses Öl erhalten, das anschließend mit Diisobutylaluminiumhydrid umgesetzt wird.

**Beispiel 14**

14α,17α-Ethano-16ß-methyl-1,3,5(10)-estratrien-3,17ß-diol (15)

2,22 g (6,02 mMol) (14) wurden mit 84,4 ml (101,2 mMol) Diisobutylaluminiumhydrid (1,2 molar in Toluol) 5 Stunden unter Inertgas bei Rückfluß gehalten. Die Reaktionsmischung wurde analog zu (10) aufgearbeitet. Nach Trocknung wurden 1,70 g Feststoff erhalten, der nach Chromatographie an Kieselgel mit Toluol → Toluol/Essigester (4:1) 355 mg (15) vom Schmelzpunkt 233 °C ergab.
$[\alpha]_D^{20}$ +82,0° (C 0,1; $CHCl_3$).

**Beispiel 15**

14α,17α-Ethano-1,3,5(10)-estratrien-3,17ß-diol (16)

200 mg (0,675 mMol) (6) in 25 ml Ethanol wurden in Gegenwart von 50 mg Pd-C (10%) bei Normaldruck hydriert. Nach Filtration und Verdampfung des Lösungsmittels wurden 200 mg (16) vom Schmelzpunkt 227-230 °C als Rohprodukt erhalten.

**BEISPIEL 16**

17ß-Acetoxy-16α-(2,2-dibromvinyl)-14α,17α-ethano-3-methoxy-1,3,5(10)-estratrien (17)

Zu 7,3 g 7 und 30,0 g Triphenylphosphin in 583 ml Methylenchlorid werden bei -10 °C 18,9 g Tetrabromkohlenstoff gegeben. Die Reaktionsmischung wird noch 2 Stunden bei -10 °C gerührt und danach mit Natriumhydrogencarbonatlösung und Wasser gewaschen. Die organische Phase wird über Natriumsulfat getrocknet, filtriert und eingeengt. Filtration des festen Rückstandes (46,3 g) mit Essigester/Hexan (1:3) durch Kieselgel ergeben 9,8 g 17 als kristallisierendes Öl.

**BEISPIEL 17**

17ß-Acetoxy-16α-(2,2-dibromvinyl)-14α,17α-ethano-3-methoxy-1,3,5(10)-estratrien-3-ol (18)

38,1 ml einer 1 molaren Lösung von Bortribromid in Methylenchlorid werden bei -35 °C zu 9,5 g 17 in 229 ml Methylenchlorid tropfenweise gegeben. Nach 4 Stunden Rühren bei 0 °C wird die Reaktionsmischung mit Eiswasser und mit Methylenchlorid versetzt. Die organische Phase wird mit Wasser gewaschen und über Natriumsulfat getrocknet. Nach Filtration und Entfernung des Lösungsmittels werden 9,2 g 18 als hellgelber Schaum erhalten.

**BEISPIEL 18**

16α-Ethinyl-14α,17α-ethano-1,3,5(10)-estratrien-3,17ß-diol (19)

4,0 g 18 in 73 ml Tetrahydrofuran werden bei -78 °C unter Stickstoff mit 28,6 ml einer 1,6 molaren Lösung von Butyllithium in Tetrahydrofuran tropfenweise versetzt. Nach 3,5 Stunden Rühren bei -78 °C werden zur Reaktionsmischung 100 ml gesättigte Ammoniumchloridlösung gegeben. Nach Erwärmung auf Raumtemperatur wird mit Essigester extrahiert und die organische Phase mit Wasser gewaschen. Nach Trocknung über Natriumsulfat, Filtration und Verdampfung des Lösungsmittels fallen 3 g Feststoff an, der nach Chromatographie an Kieselgel mit Essigester/Hexan (1:2) 1,4 g Feststoff ergibt. Nach Umkristallisation in Isopropanol werden 380 mg 19 vom Schmelzpunkt 226-228 °C erhalten.

**Beispiel 19**

3-Methoxy-1,3,5(10),14,16-estra-pentaen-18-methyl-17-yl-acetat

2,5 g 13ß-Ethyl-3-methoxygona-1,3,5(10),14-tetraen-17-on (hergestellt nach US-Patent 3,577,410) und 1,0 g p-Toluolsulfonsäureanhydrid wurden in einer Mischung aus 25 ml Essigsäureanhydrid und 25 ml Isopropenylacetat 8 Stunden am Rückfluß erhitzt. Zur Aufarbeitung wurde die erhaltene Reaktionsmischung in Eiswasser gefüllt und dann Pyridin zugegeben. Es wurde 2 Stunden nachgerührt, in Essigester aufgenommen, mit wäßriger Natriumhydrogencarbonat- und Kochsalzlösung neutral gewaschen und über Natriumsulfat getrocknet. Abziehen des Lösungsmittels im Vakuum ergab 2,4 g Rohprodukt, dessen weitere Reinigung [HPLC, reversed phase, 600 ml Säule, Elutionsmittel Hexan/Essigester (0 → 30% Essigester)] lieferte 800 mg der Titelverbindung vom Fp. 120 °C.

**Ansprüche**

1.) 14α,17α-Ethano-estratriene der allgemeinen Formel I

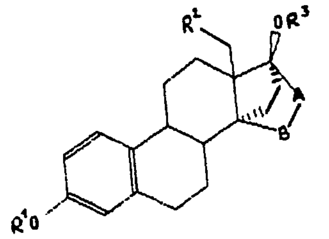

(I),

worin
$R^1$ ein Wasserstoffatom, eine Alkyl- oder Acylgruppe mit 1 bis 12 Kohlenstoffatomen,
$R^2$ ein Wasserstoffatom oder eine Methylgruppe,
$R^3$ ein Wasserstoffatom oder eine Acylgruppe mit 1 bis 12 Kohlenstoffatomen und

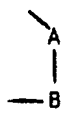 entweder 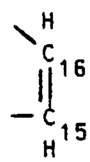 oder 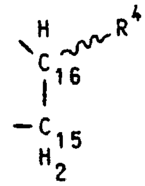

wobei $R^4$ ein α- oder ß-ständiger Alkyl- mit 1 bis 8 Kohlenstoffatomen oder ein gegebenenfalls mehrere Doppelbindungen aufweisender α,ß-Alkenyl- oder ein α,ß-Alkinylrest, beide mit 2 bis 8 Kohlenstoffatomen, ist,
bedeuten.

2.) 14α,17α-Ethano-1,3,5(10),15-estratetraen-3,17ß-diol;
14α,17α-Ethano-16α-methyl-1,3,5(10)-estratrien-3,17ß-diol;
14α,17α-Ethano-16ß-methyl-1,3,5(10)-estratrien-3,17ß-diol;
14α,17α-Ethano-3-methoxy-1,3,5(10),15-estratetraen-17ß-ol-acetat;
14α,17α-Ethano-3-methoxy-1,3,5(10)-estratrien-16α-methyl,-17ß-ol-acetat;
14α,17α-Ethano-3-methoxy-1,3,5(10)-estratrien-16ß-methyl-17ß-ol-acetat und
16α-Ethinyl-14α,17α-ethano-1,3,5(10)-estratrien-3,17ß-diol.

3.) Verfahren zur Herstellung von Verbindungen der allgemeinen Formel I

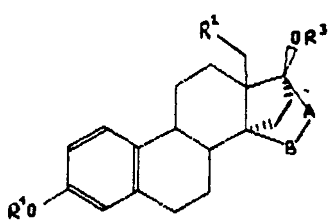

(I),

worin $R^1$, $R^2$, $R^3$ und

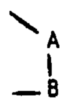

die bereits angegebene Bedeutung haben,
dadurch gekennzeichnet, daß
a) wenn

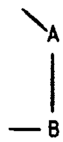 für 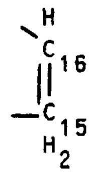

steht, eine Verbindung der allgemeinen Formel IIIa

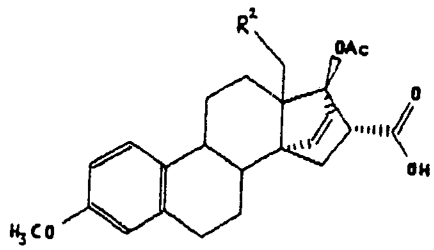

(IIIa),

worin
$R^2$ die in Formel I angegebene Bedeutung hat
zunächst hydriert und dann
zu einer Verbindung der allgemeinen Formel II'a

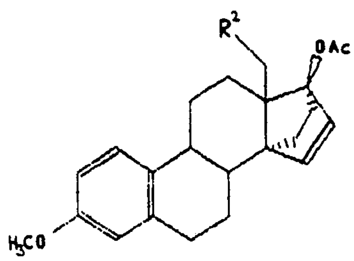

(II'a)

decarboxyliert und oxidiert wird,
b) oder wenn

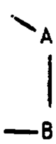 für 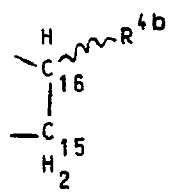

mit $R^{4b}$ in der Bedeutung einer Methylgruppe steht, eine Verbindung der allgemeinen Formel IIIb

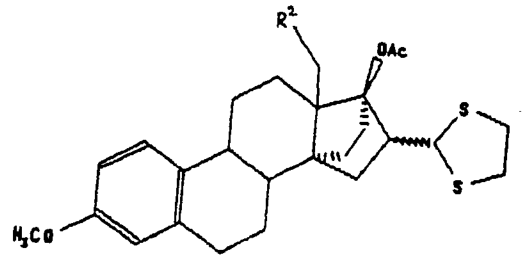

(IIIb),

worin $R^2$ die bereits angegebene Bedeutung hat, zu einer Verbindung der allgemeinen Formel II′b

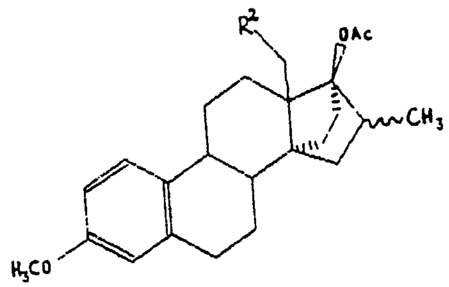

(II′b)

reduziert wird,

c) oder wenn

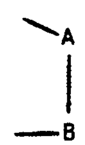

für

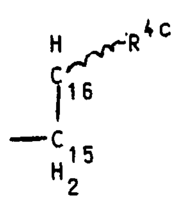

mit $R^{4c}$ in der Bedeutung eines Alkyl- oder eines, gegebenenfalls mehrere Doppelbindungen aufweisenden α,β-Alkenylrestes, beide mit 2 bis 8 Kohlenstoffatomen, steht, eine Verbindung der allgemeinen Formel IIIc

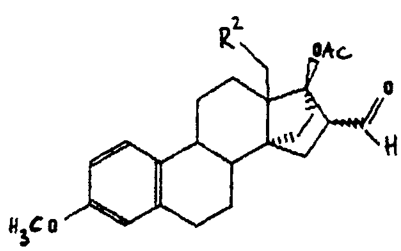

(IIIc),

worin $R^2$ die bereits angegebene Bedeutung hat, mit einem entsprechenden Wittig-Reagenz zu einer Verbindung der allgemeinen Formel II″c

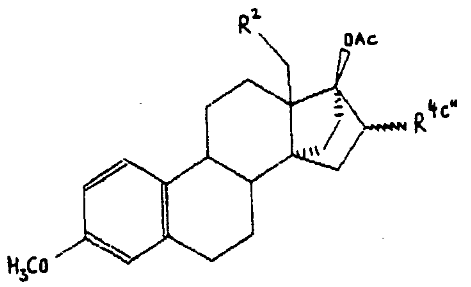

(II"c),

worin $R^{4c''}$ einen gegebenenfalls mehrere Doppelbindungen aufweisenden α,β-Alkenylrest mit 2 - 8 Kohlenstoffatomen darstellt,
umgesetzt wird,
die Verbindung der allgemeinen Formel II"c gegebenenfalls zu einer Verbindung der allgemeinen Formel II'c

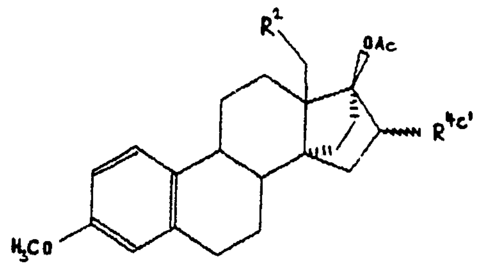

(II'c),

worin $R^{4c'}$ einen Alkylrest mit 2 bis 8 Kohlenstoffatomen darstellt, hydriert wird
d) oder wenn

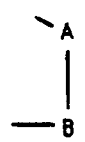

für

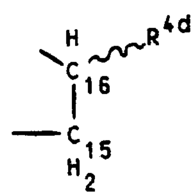

mit $R^{4d}$ in der Bedeutung eines α,β-Alkinylrestes mit 2 bis 8 Kohlenstoffatomen steht, eine Verbindung der allgemeinen Formel III c

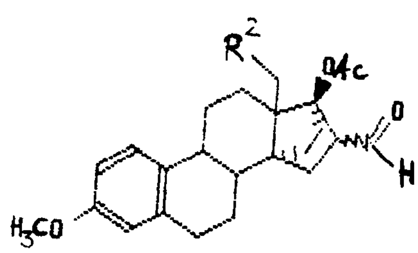

(IIIc)

in einer modifizierten Wittig-Reaktion mit einem Gemisch aus Triphenylphosan und Tetrahalogenmethan $CHal_4$, wobei Hal Brom und Jod, vorzugsweise Brom, sein kann,
zu einer Verbindung der allgemeinen Formel II"d

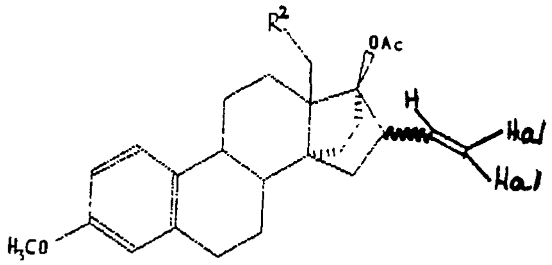

(II''d)

umgesetzt wird
und anschließend in einer Verbindung einer der allgemeinen Formeln II'a, II'b, II''c, II'c oder II''d gewünschtenfalls den 3-Methylether spaltet und
falls Verfahrensvariante d) vorliegt, nach Überführung der Verbindung der allgemeinen Formel II''d oder der entsprechenden 3-Hydroxyverbindung durch Abspaltung von HHal mit einer Base und Halogen-/Wasserstoffaustausch und gegebenenfalls nach Kupplung der entstandenen H-Ethinylverbindung mit einem 1 bis 6 Kohlenstoffatome aufweisenden linearen Alkylierungsreagenz in eine Verbindung der allgemeinen Formel II'd

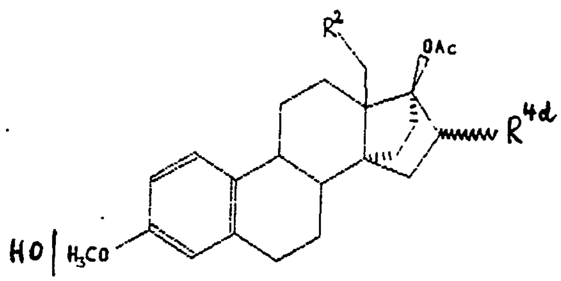

(II'd),

worin $R^{4d}$ die bereits angegebene Bedeutung hat und anschließend gewünschtenfalls die freigesetzte 3-Hydroxygruppe wieder verethert und/oder die 17-Acetoxygruppe verseift, gegebenenfalls die 3-und 17-Hydroxygruppen wieder verestert und gegebenenfalls eine so -erhaltene 3,17-Diacyloxyverbindung selektiv zur 3-Hydroxy-17-acyloxyverbindung verseift.

4.) Pharmazeutische Präparate gekennzeichnet durch einen Gehalt mindestens einer der Verbindungen gemäß den Ansprüchen 1 und 2 sowie eines in der Galenik üblichen Trägermaterials.

5.) Verwendung der Verbindungen der Ansprüche 1 und 2 zur Fertilitätskontrolle der Frau.

6.) Verwendung der Verbindungen der Ansprüche 1 und 2 zur Behandlung von Estrogen-Mangelkrankheiten der Frau.

7.) Verbindungen der allgemeinen Formel IV

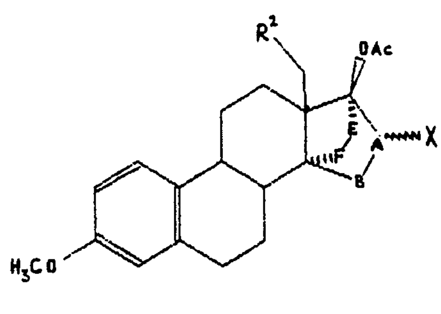

(IV),

worin
$R^2$ für ein Wasserstoffatom oder eine Methylgruppe steht und entweder
X eine $\alpha$-ständige Carbaldehydgruppe

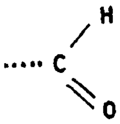

und
A-B eine C-C-Einfachbindung sowie E-F eine C-C-Doppel- oder C-C-Einfachbindung
oder
X eine ß-ständige Carbaldehydgruppe

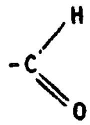

und
A-B und E-F zwei C-C-Doppel- oder zwei C-C-Einfachbindungen bedeuten.

Europäisches Patentamt

# EUROPÄISCHER RECHERCHENBERICHT

Nummer der Anmeldung

EP 89 25 0082

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | WO-A-8 801 275 (SCHERING AG)<br>* Insgesamt *<br>--- | 1-6 | C 07 J 53/00<br>A 61 K 31/56 |
| D,X | JOURNAL OF THE CHEMICAL SOCIETY, CHEMICAL COMMUNICATIONS, Nr. 6, März 1986, Seiten 451-453, Cambridge, GB; J. BULL: "Cycloaddition Route to 14alfa-Formylestrone and Derived 14alfa-Substituted Products"<br>* Seite 452, Verbindungen 2,3a *<br>--- | 1,2 | |
| P,X | LIEBIGS ANNALENDER CHEMIE, Nr. 2, Februar 1989, Seiten 151-158, Weinheim, DE; S. SCHOLZ: "Synthese von 14,17-überbrückten 11beta-Arylsteroiden"<br>* Insgesamt *<br>----- | 1,4,5,6 | |

RECHERCHIERTE SACHGEBIETE (Int. Cl.5)

C 07 J 53/00
A 61 K 31/00

Der vorliegende Recherchenbericht wurde für alle Patentansprüche erstellt

| Recherchenort | Abschlußdatum der Recherche | Prüfer |
|---|---|---|
| DEN HAAG | 19-01-1990 | WATCHORN P.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)